# EUROPÄISCHE PATENTSCHRIFT

(11) **EP 3 104 927 B1**
(45) Veröffentlichungstag und Bekanntmachung des Hinweises auf die Patenterteilung: **23.12.2020**
(21) Anmeldenummer: 15703944.7
(22) Anmeldetag: 03.02.2015
(51) Int. Cl.: A61M 37/00, A61B 17/20, A61B 5/15

(54) **MIKRONADELSYSTEM UND VERFAHREN SEINER HERSTELLUNG**
MICRO-NEEDLE SYSTEM AND METHOD FOR PRODUCING THE SAME
SYSTÈME DE MICRO-AIGUILLE ET SON PROCÉDÉ DE FABRICATION

(30) Priorität: 10.02.2014 EP 14154481
(43) Veröffentlichungstag der Anmeldung: 21.12.2016
(73) Patentinhaber: LTS Lohmann Therapie-Systeme AG, 56626 Andernach (DE)
(72) Erfinder: SCHERR, Sebastian, 56335 Neuhäusel (DE); HEUSER, Karsten, 53498 Bad Breisig (DE)
(86) Internationale Anmeldenummer: PCT/EP2015/052150
(87) Internationale Veröffentlichungsnummer: WO 2015/117938

(56) Entgegenhaltungen:
- WO-A1-2005/016440
- WO-A1-2012/100002
- WO-A2-03/048031
- WO-A2-2008/067290
- US-A1- 2007 161 964
- US-A1- 2013 158 482
- US-B1- 6 537 264

## Beschreibung

Die Erfindung betrifft ein Verfahren zur Herstellung eines Mikronadelsystems, wobei aus einer Platine ein Steggitter mit einer Vielzahl von durch jeweils drei Durchbrüche definierten Gitterknoten und mit Steggitterkeilen hergestellt wird sowie ein Mikronadelsystem mit einer Platine und mit einer Vielzahl von durch ein Steggitter umlaufend begrenzten Öffnungen, wobei an jeder Öffnung mindestens ein Steggitterkeil mit einer von der Platine abstehenden, zumindest annähernd normal zur Platine orientierten nadelförmigen Spitze angeordnet ist, wobei jeder Steggitterkeil eine Biegezone mit einer parallel zur Ebene des Steggitters orientierten Biegelinie umfasst und wobei die Anzahl der nadelförmigen Spitzen mindestens dem Dreifachen der Anzahl der Öffnungen entspricht.

Nach der US 2003/0199810 A1 wird bei der Herstellung eines Mirkronadelsytems ein von einem großen Stempel belasteter elastomerer Werkstoff eingesetzt, der die jeweils eine Spitze pro Durchbruch verbiegt. Vor der Bearbeitung zeigen alle Spitzen in die gleiche Richtung. Tritt bei der Applikation eine Querbelastung auf, können die Nadeln abknicken. Das Mikronadelsystem wird unbrauchbar. Analog gilt dies für die US 2007/161964 A1, die WO 2008/067290 A2 und die WO 2012/100002 A1.

Nach der DE 697 30 971 T2, der US 6 537 264 B1 und der WO 2005/016440 A1 können an gegenüberliegenden Seiten eines Durchbruchs zueinander versetzte Nadeln angeordnet sein. Auch in dieser Ausführung kann eine Querbelastung zum Versagen des Mikronadelsystems führen.

Gemäß der US 2013/0158482 A1 werden mehrere Nadeln an einem gemeinsamen Ring angeordnet, wobei der Ring die Dicke einer einzigen Nadel hat.

Der vorliegenden Erfindung liegt die Problemstellung zugrunde, die Betriebssicherheit eines Mikronadelsystems zu erhöhen.

Diese Problemstellung wird mit den Merkmalen des Hauptanspruchs gelöst. Dazu ragen bei der Herstellung in jeden Durchbruch aus mindestens drei verschiedenen Richtungen Steggitterkeile mit nadelförmigen Spitzen hinein. Zur Herstellung von Öffnungen werden mittels eines Stempels zumindest sämtliche dieser in einen Durchbruch ragenden Steggitterkeile gebogen und ihre nadelförmigen Spitzen normal zur Ebene des Steggitters ausgerichtet.

Das so hergestellte Mikronadelsystem ist so aufgebaut, dass an jeder Öffnung mindestens zwei Steggitterkeile angeordnet sind, deren Biegelinien nichtparallel zueinander orientiert sind. Außerdem definieren im Steggitter jeweils drei dieser Öffnungen einen Gitterknoten.

Weitere Einzelheiten der Erfindung ergeben sich aus den Unteransprüchen und den nachfolgenden Beschreibungen schematisch dargestellter Ausführungsbeispiele.
Figur 1: Draufsicht einer Platine;
Figur 2: Teil-Längsschnitt von Figur 1;
Figur 3: Detail von Figur 2;
Figur 4: Platine mit Durchbrüchen;
Figur 5: Dimetrische Ansicht von Figur 4;
Figur 6: Detail von Figur 4;
Figur 7: Variante von Figur 6;
Figur 8: zweite Variante von Figur 6;
Figur 9: dritte Variante von Figur 6;
Figur 10: Herstellung der Öffnungen;
Figur 11: Draufsicht eines Stempelsystems;
Figur 12: Draufsicht eines Matrizensystems;
Figur 13: Stempel mit umgeformter Platine;
Figur 14: Dimetrische Ansicht einer Platine nach dem Umformen.

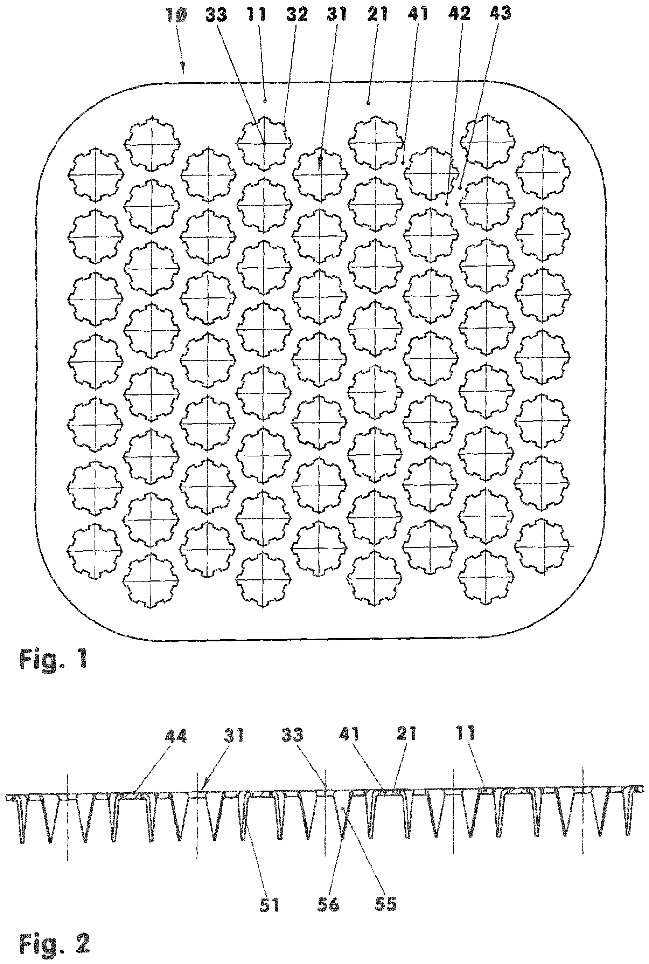

Die Figur 1 zeigt eine Draufsicht und die Figur 2 zeigt einen Längsschnitt eines Mikronadelsystems (10). Derartige Mikronadelsysteme (10) werden eingesetzt, um einen Wirkstoff aus einem hier nicht dargestellten Wirkstoffdepot durch die Haut hindurch in den Körper eines Patienten einzubringen.

Das Wirkstoffdepot ist auf einem Wirkstoffträger (11) angeordnet. Dieser Wirkstoffträger (11) umfasst eine Platine (21), die ein Steggitter (41), eine Vielzahl von Öffnungen (31) sowie eine Vielzahl von Nadeln (55) umfasst. Im Ausführungsbeispiel sind in der Platine (21) mehr als 30 Öffnungen (31) angeordnet. Jede Öffnung (31) ist von acht Nadeln (55) begrenzt. Jede Nadel (55) hat eine Spitze (56). Im dargestellten Ausführungsbeispiel haben die Öffnungen (31) eine geschlossene Kontur. Beispielsweise haben sie in der Grundform die Gestalt eines Achtecks, dessen Ecken (32) zwischen den einzelnen Nadeln (55) angeordnet sind. Die Grundform kann auch ein regelmäßiges oder unregelmäßiges Dreieck, Viereck, Sechseck, etc. sein. Auch eine Ausführung der Grundform als Kreis, Ellipse, Oval, etc. ist denkbar.

Im dargestellten Ausführungsbeispiel hat die Platine (21) eine Länge von 20 Millimetern und eine Breite von 20 Millimetern. Die Ecken sind mit einem Eckenradius von fünf Millimetern abgerundet. Die Dicke der Platine (21) beträgt 0,1 Millimeter. Die Platine ist in diesem Ausführungsbeispiel aus einem austenitischen, rost- und säurebeständigen Werkstoff hergestellt. Dies ist z.B. X5CrNi18-10 mit der Werkstoffnummer 1.4301.

In der in der Figur 1 dargestellten Platine (21) sind 67 Öffnungen (31) angeordnet. Die Summe der Querschnittsflächen aller Öffnungen (31) beträgt in diesem Ausführungsbeispiel 40 % der Grundfläche der Platine (21). Die Summe der Querschnittsflächen aller Öffnungen (31) ist damit größer als 30 % der Grundfläche der Platine (21).

Die Öffnungen (31) sind vom Steggitter (41) umgeben. Jeweils drei Öffnungen (31) bestimmen einen Gitterknoten (42). In jedem Gitterknoten (42) sind drei Stege (43) t-förmig miteinander verbunden.

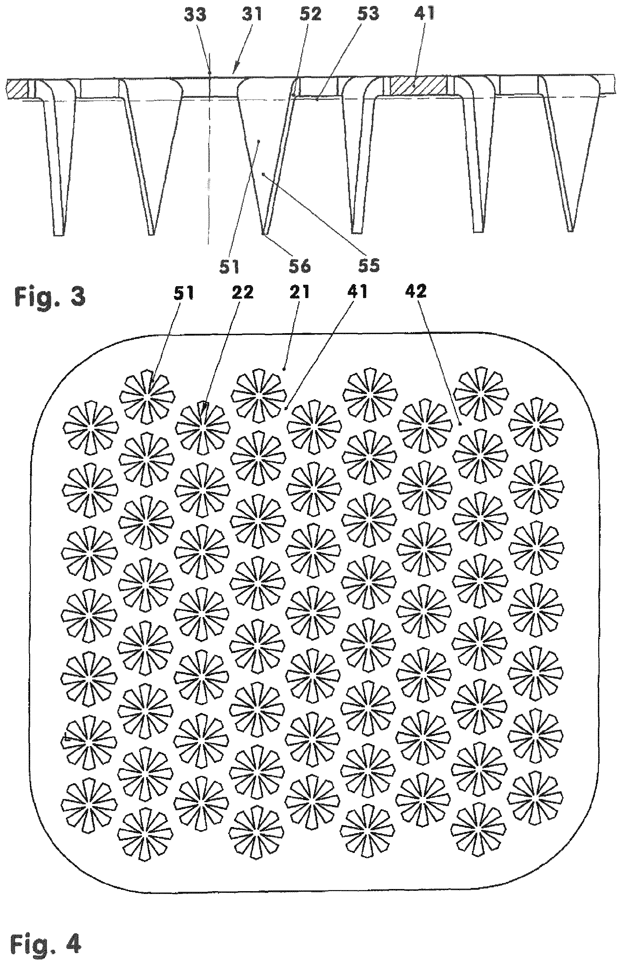

Das Steggitter (41) liegt in einer Ebene, aus der normal zu dieser Ebene Steggitterkeile (51) herausragen. In der Darstellung der Figur 2 ragen diese nach unten. Jeder Steggitterkeil (51) umfasst eine am Steggitter (41) angeschlossene Biegezone (52) und eine Nadel (55), vgl. Figur 3. Die Spitzen (56) der einzelnen Nadeln (55) sind symmetrisch zu der in Dickenrichtung der Platine orientierten Mittenlängsebene der Steggitterkeile (51) durch die Mittelachse (33) der Öffnung (31) ausgebildet. Die Dicke des einzelnen Steggitterkeils (51) entspricht der Dicke der Platine (21). Die Biegezone (52) hat einen konstanten Biegeradius, der beispielsweise der Dicke des Steggitterkeils (51) entspricht. Die Steggitterkeile (51) ragen damit mindestens um 80 % ihrer Länge aus dem Steggitter (41) heraus. Im Ausführungsbeispiel stehen die Nadeln (55) mindestens um 0,6 Millimeter aus dem Steggitter (41) hervor.

An einer Öffnung (31) sind mindestens drei Steggitterkeile (51) angeordnet. Im vorliegenden Ausführungsbeispiel ist jede der Öffnungen (31) von der gleichen Anzahl von Steggitterkeilen (51) begrenzt. Die Biegelinien (53) von mindestens zwei Steggitterkeilen (51) zeigen in unterschiedlichen Richtungen. Diese beiden Biegelinien haben einen Schnittpunkt. Im Ausführungsbeispiel haben die geraden Biegelinien von jeweils zwei Biegezonen (52) die gleiche Richtung.

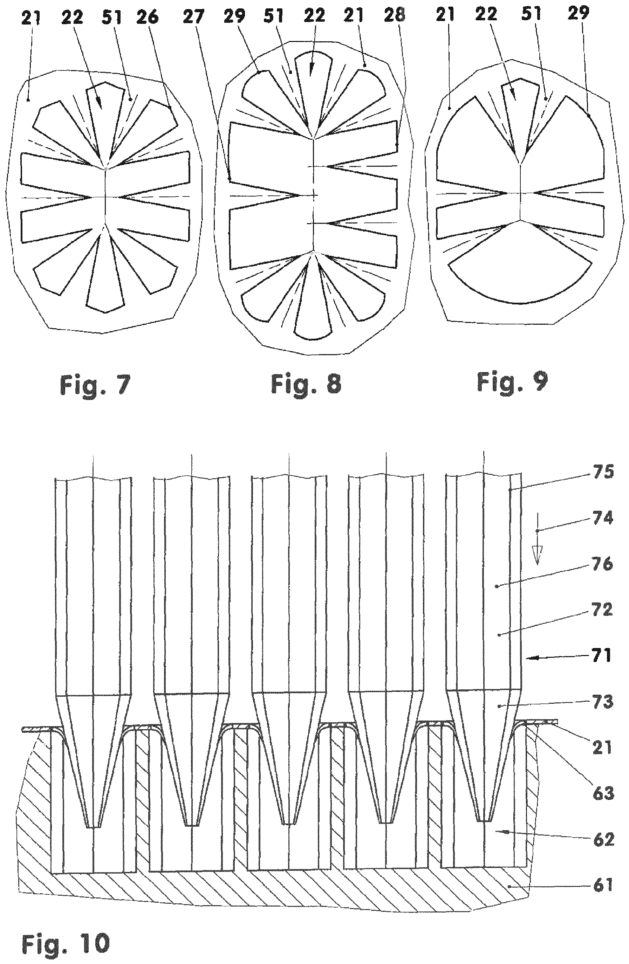

Die Herstellung des Mikronadelsystems (10) ist in den Figuren 4 - 6 und in den Figuren 10 - 14 dargestellt. Das Ausgangsmaterial ist z.B. eine ebene Platine (21) konstanter Dicke aus dem oben genannten Werkstoff. Im Ausführungsbeispiel hat sie eine quadratische Grundfläche mit abgerundeten Ecken. Die Platine (21) kann auch rund, eckig, ellipsenförmig, etc. ausgebildet sein.

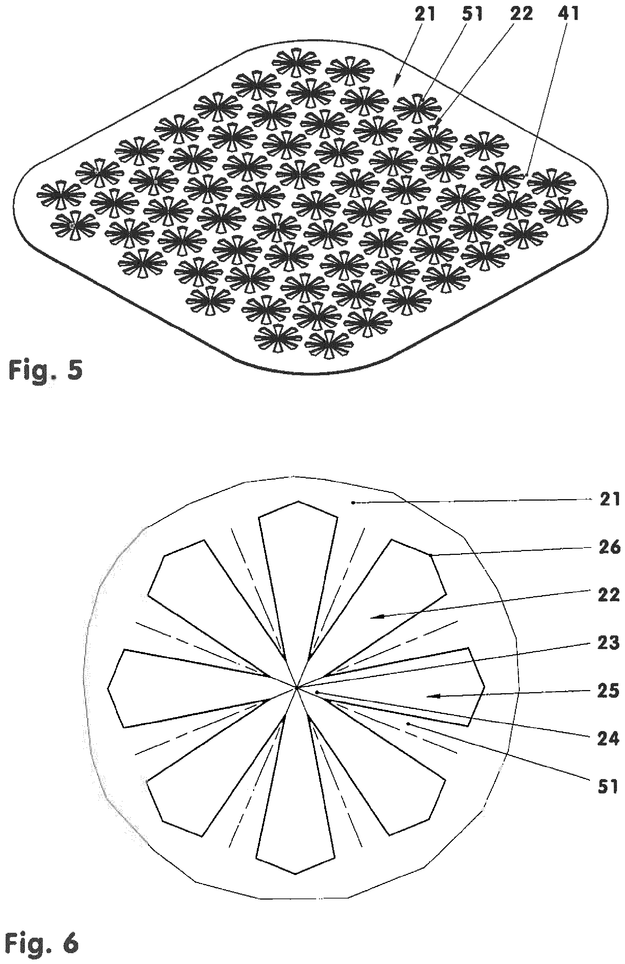

In dieses Blech werden zunächst Durchbrüche (22) eingebracht. Dies erfolgt durch Stanzen, Laserschneiden, Ätzen, etc. Die einzelnen Durchbrüche (22) sind, wie in den Figuren 4 und 5 dargestellt, beispielsweise in nebeneinanderliegenden Reihen angeordnet. Die einzelnen Reihen sind um eine halbe Teilung zueinander versetzt. Die Mittellinien (23) von jeweils drei einander benachbarten Durchbrüchen (22) bilden einen Keil mit einem gleichseitigen Dreieck als Grundfläche. Hierbei ist der Mittelpunktsabstand zweier benachbarter Durchbrüche (22) voneinander um 15 % größer als der Durchmesser des Umkreises eines einzelnen Durchbruchs (22).

Die Figur 6 zeigt das Detail eines Durchbruchs (22). Er umfasst einen zentralen Bereich (24) und z.B. acht radial zur Mittellinie angeordnete, sich nach außen aufweitende Durchbruchsabschnitte (25). Jeder dieser Durchbruchsabschnitte (25) ist symmetrisch zu einer Radialen des Durchbruchs (22), wobei jede dieser Radialen eine äußere Ecke (26) des Durchbruchs (22) schneidet.

Beispielsweise nach dem Ausstanzen bleiben von der ursprünglichen Platine (21) die Steggitter (41) und zwischen den Durchbruchsabschnitten (25) die Steggitterkeile (51) stehen. Die beispielsweise in der Draufsicht der Figur 6 dreieckigen Steggitterkeile (51) sind zwischen den Ecken (26) des Durchbruchs (22) angeordnet und ragen mit einer Länge von z.B. 0,75 Millimetern aus dem Steggitter (41) heraus. Die Breite des einzelnen Steggitterkeils (51) beträgt in dieser Draufsicht 0,3 Millimeter. In der Draufsicht beträgt in diesem Ausführungsbeispiel die Fläche aller Steggitterkeile (51) 34 % der Fläche der durch die Ecken (26) bestimmten Grundform des Durchbruchs (22). Die Fläche aller Steggitterkeile (51) beträgt zwischen 25 % und 50 % der hier z.B. achteckigen Grundform des Durchbruchs (22). Aufgrund dieser Größenverhältnisse besteht keine Gefahr der Beschädigung der Steggitterkeile (51) bei der Herstellung der Durchbrüche (22).

Die einzelnen Durchbrüche (22) sind so zueinander angeordnet, dass die Steggitterkeile (51) nicht miteinander fluchten. In der Draufsicht der Figur 4 fluchten in den einzelnen Spalten die Durchbbruchsabschnitte miteinander. In benachbarten Spalten ist ein Steggitterkeil (51) benachbart zu einem Durchbruchsabschnitt (25) angeordnet.

Die Figuren 7 - 9 zeigen Varianten der Gestalt von Durchbrüchen (22). In der Figur 7 hat die Grundform des Durchbruchs (22) zehn Ecken (26). Diese Grundform einschließlich der Steggitterkeile (51) ist symmetrisch zu einer Mittenlängsebene des Durchbruchs (22). Auch in diesem Ausführungsbeispiel ist der einzelne Steggitterkeil (51) zwischen zwei Ecken (26) des Durchbruchs (22) angeordnet. Statt der Ecken (26) können auch Bögen zwischen den Steggitterkeilen (51) angeordnet sein.

Der in der Figur 8 dargestellte Durchbruch (22) hat zwei einander gegenüber liegende Längsseiten (27, 28). An der einen Längsseite (27) ist ein einzelner Steggitterkeil (51) angeordnet. An der anderen Längsseite (28) sitzen hierzu versetzt zwei Steggitterkeile (51).

Die Figur 9 zeigt einen Durchbruch mit sechs Steggitterkeilen (51). Jeweils zwei Steggitterkeile (51) zeigen in dieser Draufsicht auf einen gemeinsamen Punkt der vertikalen Mittenlängsebene. Anstatt der Ecken (26) sind zwischen den einzelnen Steggitterkeilen (51) Bogenelemente (29) angeordnet.

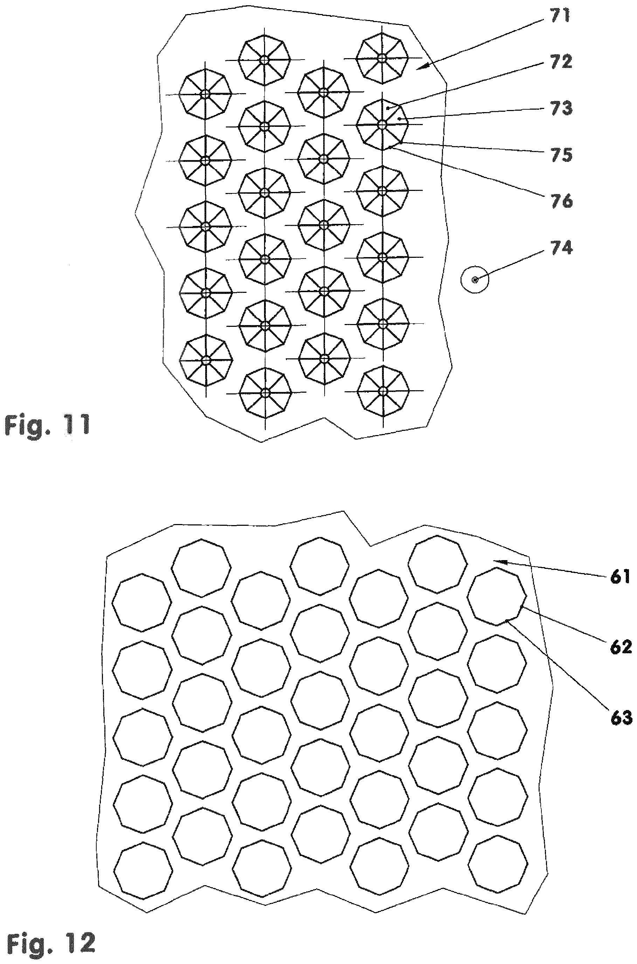

Die Platine (21) mit den Durchbrüchen (22) wird beispielsweise in eine Biegepresse eingelegt, vgl. Figur 10. Die Biegepresse umfasst ein Matrizensystem (61) und ein Stempelsystem (71). Das Matrizensystem (61), vgl. Figur 12, umfasst im Ausführungsbeispiel eine Vielzahl von Ausnehmungen (62). Die Anzahl der Ausnehmungen (62) entspricht der Anzahl der Durchbrüche (22) der Platine (21). Die Querschnittsfläche der einzelnen Ausnehmung (62) ist achteckig. Die Ausnehmung (62) weist an ihren oberen Rändern eine Abrundung (63) auf, vgl. auch Figur 10. Diese Abrundung (63) hat beispielsweise einen Radius von 0,2 Millimetern. Der innere Querschnitt der Ausnehmung (62) ist um die Abrundungen (63) kleiner als der Querschnitt der Grundform des Durchbruchs (22).

Das Stempelsystem (71), vgl. Figur 11, umfasst eine Vielzahl von Stempeln (72). Diese im Ausführungsbeispiel gleichartigen Stempel (72) haben im Ausführungsbeispiel eine achteckige Querschnittsfläche. Die Schlüsselweite ist z.B. um die zweifache Dicke der Platine (21) enger als das entsprechende Maß der Ausnehmung (62) des Matrizensystems (61). Die Spitze (73) des einzelnen Stempels (72) ist pyramidenstumpfförmig ausgebildet. Der Spitzenwinkel beträgt beispielsweise 30 Grad.

In der Biegepresse wird das Stempelsystem (71) auf das Matrizensystem (61) zubewegt. Die Platine (21) wird z.B. mittels Niederhaltern in ihrer Position gehalten. Die Stempel (72) kontaktieren die Steggitterkeile (51) und tauchen in die Matrizen (62) ein. Hierbei werden die Steggitterkeile (51) entlang der Abrundungen (63) der jeweiligen Matrize (62) umgebogen. Das Umformen kann kalt oder warm erfolgen. Die jeweilige Biegezone (52) hat eine gerade Biegelinie (53). Alle Biegelinien (53) liegen in einer gemeinsamen Ebene, die parallel ist zu der Ebene der Oberseite (44) des Steggitters (41). Die Biegelinien (53) der Steggitterkeile (51) weisen so vier verschiedene Richtungen auf. Da aufgrund der Anordnung und der Ausrichtung der Durchbrüche (22) keine einander benachbarten Bereiche verformt werden, behält das Steggitter (41) seine statische Festigkeit. Es besteht keine Knickgefahr der Steggitterkeile (51). Sowohl zum Erzeugen der Durchbrüche (22) als auch zum Umformen können geometrisch einfache Werkzeuge eingesetzt werden. Für jeden Durchbruch (22) wird beispielsweise nur ein einziger Stempel (72) eingesetzt. Mittels dieses Stempels (72) werden in einem einzigen Stempelhub aus den Steggitterkeilen (51) alle beispielsweise acht Nadeln (55) hergestellt.

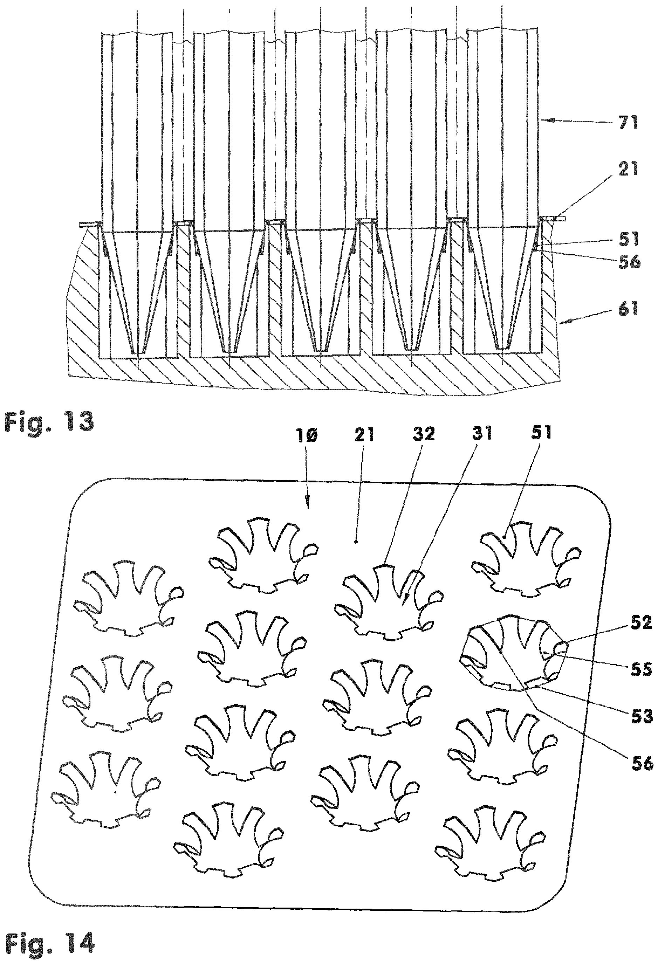

Beim weiteren Eintauchen der Stempel (72) in die Matrizen (62) werden die Steggitterkeile (51) weiter verformt, bis sie normal zur Ebene des Steggitters (41) auskragen. Die Spitzen (56) der Steggitterkeile (51) zeigen nun in die der Ebene des Steggitters (41) abgewandten Richtung. Dies ist in der Figur 13 dargestellt. Je nach Umformverfahren kann der Stempel (72) während eines vorgegebenen Zeitintervalls in dieser Position gehalten werden oder die Steggitterkeile (51) geringfügig überstrecken.

Die Steggitterkeile (51) bilden nun die Nadeln (55), die in der Darstellung der Figur 14 die Öffnungen (31) umgeben und nach unten abstehen. Beispielsweise stehen die Nadeln (55) mit einer Länge von 0,6 Millimetern aus dem Steggitter (41) hervor. Im Ausführungsbeispiel der Figur 14 sind - unter Zugrundelegung der oben genannten Abmessungen - 1,2 Nadeln pro Quadratmillimeter Platinenfläche angeordnet. Bei einem Mikronadelsystem (10), das aus der Platine (21) nach den Figuren 4 und 5 hergestellt wird, sind 1,4 Nadeln (55) pro Quadratmillimeter der Platinenfläche angeordnet. Bezogen auf die Fläche der Öffnungen (31) weisen die Ausführungsbeispiele mindestens drei Nadeln (55) und Spitzen (56) pro Quadratmillimeter des Öffnungsquerschnitts auf. Die Gesamtfläche der Öffnungen (31) beträgt beispielsweise mehr als 30 % der Grundfläche der Platine (21).

Zum Einsatz des Mikronadelsystems (10) wird dieses mit montiertem Wirkstoffdepot auf die Haut des Patienten aufgesetzt und in diese hineingedrückt. Hierbei leitet der Anwender im Wesentlichen eine in Richtung der Nadeln (55) orientierte Kraft ein. Die Nadeln (55) überwinden den Widerstand der oberen Hautschichten und dringen in die Haut ein. Beispielsweise können Widerstände der Haut und/oder eine schiefe Kraftaufbringung durch den Anwender zu Scherkräften auf die Nadeln (55) führen.

Aufgrund der unterschiedlichen Richtungen der Biegelinien (53) der einzelnen Nadeln (55) besteht beim Einsatz des beschriebenen Mikronadelsystems (10) keine Gefahr des Abknickens oder des Verbiegens der Nadeln (55). Alle Nadeln (55) dringen damit in die Haut des Patienten ein.

Nach der Applikation des Mikronadelsystems (10) dringt der Wirkstoff durch die Öffnungen (31) hindurch in die zwischen den Mikronadeln (55) gespannte Haut. Hierbei ist der Volumenstrom des Wirkstoffs umso größer, je größer das Verhältnis der Gesamtfläche der Öffnungen (31) zur Grundfläche der Platine (21) ist. Beispielsweise steigt mit einem steigenden Verhältnis der Volumenstrom proportional hierzu. Aufgrund der Anordnung der Nadeln (55) entlang geschlossener Konturen kann bei gleicher Anzahl Nadeln (55) die Fläche des Steggitters (41) klein im Verhältnis zur Gesamtfläche der Öffnungen (31) gewählt werden. Der Faktor kann z.B. kleiner als 2,5 sein.

Zur Herstellung des Mikronadelsystems (10) sind nur zwei Arbeitsgänge erforderlich. In beiden Arbeitsgängen führen die Werkzeuge jeweils eine einzige Hubbewegung durch. Das Mikronadelsystem (10) ist somit schnell und problemlos herstellbar. Es ist damit für eine Massenfertigung geeignet.

Es ist auch denkbar, die verschiedenen genannten Ausführungsformen miteinander zu kombinieren.

### Bezugszeichenliste:

- 10: Mikronadelsystem
- 11: Wirkstoffträger

- 21: Platine
- 22: Durchbrüche
- 23: Mittellinien
- 24: zentraler Bereich
- 25: Durchbruchsabschnitte
- 26: Ecke
- 27: Längsseite
- 28: Längsseite
- 29: Bogenelemente

- 31: Öffnungen
- 32: Ecken
- 33: Mittelachse

- 41: Steggitter
- 42: Gitterknoten
- 43: Stege
- 44: Oberseite

- 51: Steggitterkeile
- 52: Biegezonen
- 53: Biegelinien

- 55: Nadeln, Mikronadeln
- 56: Spitzen, nadelförmige Spitzen

- 61: Matrizensystem
- 62: Ausnehmungen, Matrizen
- 63: Abrundung
- 71: Stempelsystem
- 72: Stempel
- 73: Stempelspitze
- 74: Hubrichtung
- 75: Längskanten
- 76: Stempelfläche

## Patentansprüche

1. Verfahren zur Herstellung eines Mikronadelsystems (10),
- wobei aus einer Platine (21) ein Steggitter (41) mit einer Vielzahl von durch jeweils drei Durchbrüche (22) definierten Gitterknoten (42) und mit Steggitterkeilen (51) hergestellt wird, und
- in jeden Durchbruch (22) aus mindestens drei verschiedenen Richtungen Steggitterkeile (51) mit nadelförmigen Spitzen (56) hineinragen und
- zur Herstellung von Öffnungen (31) mittels eines Stempels (72) zumindest sämtliche dieser in einen Durchbruch (22) ragenden Steggitterkeile (51) gebogen werden und ihre nadelförmigen Spitzen (56) normal zur Ebene des Steggitters (41) ausgerichtet werden.

2. Verfahren zur Herstellung eines Mikronadelsystems (10) nach Anspruch 1,
**dadurch gekennzeichnet,**
**dass** der Stempel (72) in seiner Hubrichtung (74) orientierte Längskanten (75) aufweist, wobei jeweils zwei Längskanten (75) eine ebene Stempelfläche (76) begrenzen.

3. Verfahren zur Herstellung eines Mikronadelsystems (10) nach Anspruch 1,
**dadurch gekennzeichnet,**
**dass** die Durchbrüche (22) aufweisende Platine (21) auf einem Matrizensystem (61) mit mindestens einer Ausnehmung (62) mit polygonalem Querschgnitt aufliegt.

4. Mikronadelsystem (10) mit einer Platine (21) und mit einer Vielzahl von durch ein Steggitter (41) umlaufend begrenzten Öffnungen (31),
wobei an jeder Öffnung (31) mindestens ein Steggitterkeil (51) mit einer vom Steggitter (41) abstehenden, zumindest annähernd normal zur Ebene des Steggitters (41) orientierten nadelförmigen Spitze (56) angeordnet ist,
wobei jeder Steggitterkeil (51) eine Biegezone (52) mit einer parallel zur Ebene des Steggitters (41) orientierten Biegelinie (53) umfasst und
wobei die Anzahl der nadelförmigen Spitzen (56) mindestens dem Dreifachen der Anzahl der Öffnungen (31) entspricht,
und wobei,
- an jeder Öffnung (31) mindestens zwei Steggitterkeile (51) angeordnet sind, deren Biegelinien (53) nichtparallel zueinander orientiert sind und
- im Steggitter (41) jeweils drei dieser Öffnungen (31) einen Gitterknoten (42) definieren.

5. Mikronadelsystem (10) nach Anspruch 4,
**dadurch gekennzeichnet,**
**dass** die Gesamtquerschnittsfläche der Öffnungen (31) mindestens 30 % der Platinengrundfläche beträgt.

6. Mikronadelsystem (10) nach Anspruch 4,
**dadurch gekennzeichnet,**
**dass** die Biegelinien (53) jeweils zweier benachbarter Steggitterkeile (51) sich in genau einem Punkt schneiden.

7. Mikronadelsystem (10) nach Anspruch 4,
**dadurch gekennzeichnet,**
**dass** alle nadelförmigen Spitzen (56) Teile von Nadeln (55) sind, die zumindest annähernd die gleiche Länge aufweisen.

## Claims

1. Method for producing a microneedle system (10),
- a web grid (41) with a multiplicity of grid nodes (42) which are defined by way of in each case three apertures (22) and with web grid wedges (51) being produced from a board (21), and
- web grid wedges (51) with needle-shaped points (56) protruding into every aperture (22) from at least three different directions, and
- at least all said web grid wedges (51) which protrude into an aperture (22) being bent by means of a ram (72) in order to produce openings (31), and their needle-shaped points (56) being oriented perpendicularly with respect to the plane of the web grid (41).

2. Method for producing a microneedle system (10) according to Claim 1, **characterized in that** the ram (72) has longitudinal edges (75) which are oriented in its stroke direction (74), in each case two longitudinal edges (75) delimiting a planar ram face (76).

3. Method for producing a microneedle system (10) according to Claim 1, **characterized in that** the board (21) which has apertures (22) lies on a matrix system (61) with at least one recess (62) with a polygonal cross section.

4. Microneedle system (10) having a board (21) and having a multiplicity of openings (31) which are delimited peripherally by way of a web grid (41), at least one web grid wedge (51) with a needle-shaped point (56) which projects from the web grid (41) and is oriented at least approximately perpendicularly with respect to the plane of the web grid (41) being arranged at each opening (31), each web grid wedge (51) comprising a bending zone (52) with a bending line (53) which is oriented parallel to the plane of the web grid (41), and the number of needle-shaped points (56) corresponding at least to three times the number of openings (31), and
- at least two web grid wedges (51) being arranged at each opening (31), the bending lines (53) of which web grid wedges (51) are not oriented parallel to one another, and
- in each case three of said openings (31) defining a grid node (42) in the web grid (41).

5. Microneedle system (10) according to Claim 4, **characterized in that** the overall cross-sectional area of the openings (31) is at least 30% of the board base area.

6. Microneedle system (10) according to Claim 4, **characterized in that** the bending lines (53) of in each case two adjacent web grid wedges (51) intersect at precisely one point.

7. Microneedle system (10) according to Claim 4, **characterized in that** all needle-shaped points (56) are parts of needles (55) which have at least approximately the same length.

## Revendications

1. Procédé de fabrication d'un système de micro-aiguilles (10),
- une grille de nervures (41) pourvue d'une multitude de nœuds de grille (42), définis chacun par trois passages (22), et de coins de grille de nervures (51) étant réalisée à partir d'une plaque (21), et
- des coins de grille de nervures (51) pourvus de pointes (56) en forme d'aiguille faisant saillie dans chaque passage (22) depuis au moins trois directions différentes et
- au moins tous ces coins de grille de nervures (51), qui font saillie dans un passage (22), étant pliés au moyen d'une matrice (72), et leurs pointes (56) en forme d'aiguille étant orientées perpendiculairement au plan de la grille de nervures (41), afin de réaliser des ouvertures (31).

2. Procédé de fabrication d'un système de micro-aiguilles (10) selon la revendication 1,
**caractérisé en ce que**
la matrice (72) comporte des bords longitudinaux (75) orientés dans sa direction de course (74), deux bords longitudinaux (75) délimitant à chaque fois une surface de matrice plane (76).

3. Procédé de fabrication d'un système de micro-aiguilles (10) selon la revendication 1,
**caractérisé en ce que**
la plaque (21) comportant les passages (22) est en appui sur un système matriciel (61) comprenant au moins un évidement (62) de section transversale polygonale.

4. Système de micro-aiguilles (10) comprenant une plaque (21) et une multitude d'ouvertures (31) délimitées circonférentiellement par une grille de nervures (41), au moins un coin de grille de nervures (51), pourvu d'une pointe (56) en forme d'aiguille qui fait saillie de la grille de nervures (41) et qui est orientée au moins à peu près perpendiculairement au plan de la grille de nervures (41), étant disposé au niveau de chaque ouverture (31),
chaque coin de grille de nervures (51) comprenant une zone de pliage (52) pourvue d'une ligne de pliage (53) orientée parallèlement au plan de la grille de nervures (41), et
le nombre de pointes (56) en forme d'aiguille correspondant au moins au triple du nombre d'ouvertures (31), et
- au moins deux coins de grille de nervures (51), dont les lignes de pliage (53) ne sont pas orientées parallèlement l'une à l'autre, étant disposés au niveau de chaque ouverture (31) et
- trois de ces ouvertures (31) définissant à chaque fois un nœud de grille (42) dans la grille de nervures (41) .

5. Système de micro-aiguilles (10) selon la revendication 4,
**caractérisé en ce que**
l'aire totale de la section transversale des ouvertures (31) représente au moins 30 % de la surface de base de la plaque.

6. Système de micro-aiguilles (10) selon la revendication 4,
**caractérisé en ce que**
les lignes de pliage (53) de deux coins de grille de nervures (51) adjacents se coupent exactement en un point.

7. Système de micro-aiguilles (10) selon la revendication 4,
**caractérisé en ce que**
toutes les pointes (56) en forme d'aiguille sont des parties d'aiguilles (55) qui ont au moins à peu près la même longueur.
